# EUROPEAN PATENT SPECIFICATION

(11) **EP 1 348 765 B1**
(45) Date of publication and mention of the grant of the patent: **24.05.2006**
(21) Application number: 02252302.1
(22) Date of filing: 28.03.2002
(51) Int. Cl.: C12P 17/10, C12P 41/00, C12N 11/16, C12R 1/01, C12R 1/07, C12R 1/185

(54) **Process for the preparation of optically active azabicyclo heptanone derivatives**
Verfahren zur Herstellung von optisch aktiven Azabicycloheptanone-Derivaten
Procédé pour la préparation de dérivés optiquement actifs de l'azabicycloheptanone

(43) Date of publication of application: 01.10.2003
(73) Proprietor: COUNCIL OF SCIENTIFIC AND INDUSTRIAL RESEARCH, New Delhi 110 001 (IN)
(72) Inventor: Joshi, Rohini Ramesh, Pune 411 008, Maharashtra (IN); Prabhune, Asmita Ashutosh, Pune 411 008, Maharashtra (IN); Joshi, Ramesh Anna, Pune 411 008, Maharashtra (IN); Gurjar, Mukund Keshav, Pune 411 008, Maharashtra (IN)
(74) Representative: Manaton, Ross Timothy

(56) References cited:
- EP-A- 0 424 064
- WO-A-99/10519
- TAYLOR S J ET AL: "Novel screening methods--the key to cloning commercially successful biocatalysts." BIOORGANIC & MEDICINAL CHEMISTRY. ENGLAND OCT 1999, vol. 7, no. 10, October 1999 (1999-10), pages 2163-2168, XP002216422 ISSN: 0968-0896
- MAHMOUDIAN M ET AL: "A practical enzymatic procedure for the resolution of N-substituted 2-azabicyclo[2.2.1]hept-5-en-3-one" TETRAHEDRON: ASYMMETRY, ELSEVIER SCIENCE PUBLISHERS, AMSTERDAM, NL, vol. 10, no. 6, 26 March 1999 (1999-03-26), pages 1201-1206, XP004164871 ISSN: 0957-4166
- NAKANO H ET AL: "Lipase-Catalyzed Resolution of 2-Azabicyclo[2.2.1]hept-5-en-3-ones" TETRAHEDRON: ASYMMETRY, ELSEVIER SCIENCE PUBLISHERS, AMSTERDAM, NL, vol. 7, no. 8, 1 August 1996 (1996-08-01), pages 2381-2386, XP004048294 ISSN: 0957-4166

## Description

### Field of the invention

The present invention relates to a process for the preparation of an optically active azabicyclo heptanone derivative of general formula III 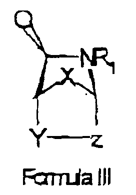 wherein R₁=H, X=CH₂ Y-Z = -CH=CH- from racemic γ-lactam of formula (I) 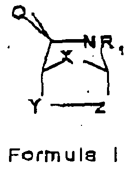 Wherein R₁ is H or COR₂ (R₂ is C ₁₋₄ alkyl, C₁₋₄ alkoxy, aryl or aryloxy)
X is O or CHR₃ (R₃ is F, OH, Br or H)
Y-Z=-CH=CH-, CH₂-CH₂.

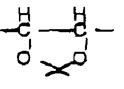

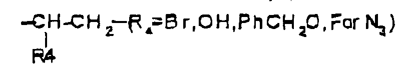

Optically active azabicyclo heptanone derivatives of general formula III wherein R₁=H, X = CH₂, Y-Z = CH = CH₂ are useful as intermediates in the synthesis of antiviral agents.

### Background of the invention

Carbocyclic analogues of purines are known as antiviral and anti neoplastic agents. For example the compound of formula (II) is described as having potent activity against human immunodeficiency virus (HIV) and Hepatitis B virus (HBV) (EP 0434450). 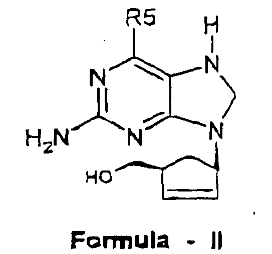 R₅ =Cycloproppylamino, =N-Cyclopropyl, N-Methylamine

Prior art discloses the preparation of 9-substituted-2-amino purines starting from a pyrimidine compound, coupling with enantiomerically pure sugar/carbocyclic analogues residue and cyclization to form the imidazole ring followed by introduction of suitable 6-substituent (WO 95/21161). Carbovir and known analogues are prepared from the known γ-lactam(Vince lactam)2-azabicyclo[2,2,1]hept-5-en-3-one (Formula I) wherein X is -CH₂, - Y-Z- is -CH=CH- and R₁ is H.

Prior art indicates that the final product or any intermediate or starting material may be resolved by known methods or the racemic mixture of the product may be enzymatically converted to chirally pure compound. The γ-lactam can be prepared by reacting cyclopentadiene with tosylcyanide,(Vince J. Org. Chem. 1978, 43, 2311).

There are several synthetic pathways where chemical resolution into the enantiomer has been effected but the enzymatic resolution of γ-lactam will be the most economical commercial process. γ-lactamase methodology has been reported based on enantio complementary biotransformation. Enzymatic resolution of bicyclic lactam using whole cell cultures ENZA1 and ENZA2 has been reported to give both the optical forms of lactam (S.V. Teylor, J.C.S. Chem. Comm., 1121, 1990, Tet. Assy., 4, 1117-1128). The detailed process has been described in patent (EP 0424064). The racemic lactam was treated with ENZA-1/2 cell free extract at 30°C with shaking for 14 days. The crude (+)/ (-) lactam was isolated by extraction with dichloromethane purified by column chromatography on silica geL The (+) lactam obtained with 88% ee and (-) lactam 98% ee.

Enzymatic resolution of N-Acyl bicyclic lactam using acylase has been described in patent (WO 00/03032) in 31% yield with 98% ee. The conversion of the optically active N-Acetyl-lactam to(+) /(-) lactam is tedious.

Taylor, S.J.C. *et al* ("Novel Screening Methods - The Key to Cloning Commercially Successful Biocatalysts" Bioorg. & Medic. Chem. 7 (1999) 2163-2168) describe the problems associated with biocatalytic resolution of 2-aza-bicyclo[2.2.1]hept-5-en-3-one using *Pseudomonas cepaecia* and propose *Comamonas acidovorans* as an alternative.

WO 99/10519 relates to the enantiomeric resolution of N-protected (±)2-azabicylco[2.2.1]hept-5-en-3-one using acylase enzymes, in particular enzymes derived from *Bacillus sp.*

Nakano, H. *et al* ("Lipase-Catalyzed Resolution of 2-Azabicyclo [2.2.1] hept-5-en-3-ones" Tetrahedron Asymmetry 7 (1996) 2381-2386) report the lipase-catalysed enantioselective transesterification or hydrolysis of *N*-hydroxymethyl-2-azabicyclo[2.2.1]hept-5-en-3-one to produce chiral 2-azabicylco[2.2.1]hept-5-en-3-ones.

Mahmoudian *et al* ("A Practical Enzymatic Procedure for the Resolution of *N-*substituted 2-azabiclyco[2.2.1]hept-5-en-3-one" Tetrahedron Asymmetry 10 (1999) 1201-1206) refer to existing techniques for the resolution of racemic 2-azabicyclo[2.2.1]hept-5-en-3-one using γ-lactamase containing microorganisms such as *Pseudomonas solanacearum* and *Rhodococcus sp.,* and report the screening of various esterases, lipases and proteases of microbial and mammalian origin for ability to hydrolyse the lactam bond of (±)2-*tert*-butyl 3-oxo-2-azabicyclo[2.2.1]hept-5-ene-2-carboxylate.

The prior art methods to the cyclopentane moiety of carbocyclic nucleosides starting from non-carbohydrate synthons or readily available meso compounds generally involve a number of steps, are often difficult to perform and provide poor yields making practical scale-up of these processes difficult and uneconomical

### Objects of the invention

The main object of the present invention is to provide a process for the preparation of an optically active azabicyclo heptanone derivative which obviates the drawbacks of the prior art and use cheaper and easily available microbial whole cell enzyme.

It is another object of the invention to provide a process for the preparation of (-) 2-Azabicyclo[2,2,1]-hept-5-ene-3-one formula (III) which is economical and efficient.

### Summary of the invention

The present invention enables the preparation of optically active azabicyclo heptanone derivatives using lactamases that will react with racemic γ-lactam of formula (I) to give a single enatiomer of lactam (III) and the corresponding ring opened compound of formula (IV) in an enatiomerically pure form.

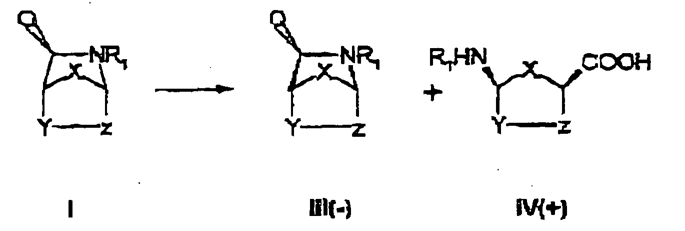

Accordingly, the present invention provides a process for the preparation of an optically active azabicyclo heptanone derivative of the general formula (III) wherein R₁=H, X=CH₂ Y-Z = -CH=CH- 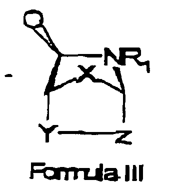 which comprises contacting (±) 2 - azabicyclo [2.2.1] hept -5-en-3-one of formula (I) 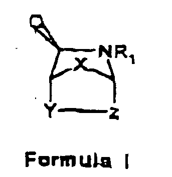 Wherein R₁=H, COR₂ (R₂=C₁₋₄ alkyl, C₁₋₄ alkoxy, aryl, aryloxy)
X=O, CHR₃ (R₁=F,OH,Br,H)
Y-Z=-CH=CH-,CH₂-CH₂, 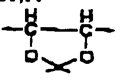 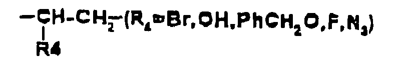 with whole cell Kluyvera Citrophila (ATCC No. 21285) or with a cell extract or lactamase enzyme extracted from *Kluyvera Citrophila* (ATCC No. 21285), in a buffer containing organic solvent, extracting the mixture with an organic solvent, separating the organic layer and removing the solvent to obtain the product.

In one embodiment of the invention the buffer used is selected from the group consisting of phosphate buffer (0.5 M - 0.1 M, 6-8 pH), citrate buffer (0.05 M - 0.1 M 6-7.5 pH) and Tris buffer (0.05 M - 0.2 M, 7-8 pH).

In another embodiment of the invention the buffer used comprises phosphate buffer (0.2 M, 7.4 pH).

In another embodiment of the invention the organic solvent used for the reaction along with buffer is selected from the group consisting of alcohols, alkyl acetates, ketones and sulfoxides.

In a further embodiment of the invention, the organic solvent is selected from the group consisting of methanol, ethanol, butanol, ethyl acetate, acetone, dimethyl sulfoxide and dimethylformamide.

In a further embodiment of the invention the organic solvent comprises acetone.

In another embodiment of the invention the percent of organic solvent used for the reaction along with buffer is in the range of 5% to 50% (v/v).

In another embodiment of the invention the percent of organic solvent used for the reaction along with buffer comprises is 10% (v/v).

In another embodiment of the invention the solvent used for extraction comprises a chlorinated solvent selected from the group consisting of chloroform, ethylene dichloride, methylene dichloride and an alkyl acetate.

In another embodiment of the invention, the alkyl acetate used as the solvent for extraction comprises ethyl acetate.

In another embodiment of the invention the solvent used for extraction comprises methylene chloride.

In another embodiment, the compound of Formula I is contacted with the enzyme or (whole) cell at a temperature in the range of 25 - 30°C.

In another embodiment, the compound of Formula I is contacted with the enzyme or (whole) cell for a period in the range 10 to 24 hours, more preferably 14 to 24 hours.

In a feature of the invention the chemical yield (-) 2-Azabicyclo [2,2,1]-hept-5-ene-3-one is 39% and optical purity is 98%.

### Detailed description of the invention

The process of the present invention is highly efficient and maximises cost effectiveness by fast resolution to provide (-) lactam enantiomer, an important starting material for the production of the anti HIV agent (-) Carbovir and Abacavir.

The process of the present invention is described herein below with references to following examples, which are illustrative only and should not be construed to limit the scope of the present invention in any manner.

### Example 1

This example describes general procedure for cell biomass preparation (whole cell)/enzyme pre-inoculum (5-10 ml) was prepared by growing microorganism in a medium containing yeast extract (0.5%), peptone (1%), sodium chloride (0.2%), sodium glutamate (0.5%) and phenyl acetic acid (15 mM) at pH 7.2-7.3 for 24 hr with shaking at 150rpm. This was subsequently transferred to 1 Lt. flask containing 300 ml above mentioned growth medium and incubated at 28-30°C for 24 hours on rotary shakers (150 rpm). The grown cells were separated by centrifuge washed with phosphate buffer pH 6.8 and biomass was used for the reaction.

### Example 2

### General procedure for enantioselectsive hydrolysis of (±)-2-azabicyclo (2,2,1) hept-5-en-3-one, (Vince's lactam) (1).

0.1 g (0.00092 mole parts) of (±)-2-azabicyclo[2,2,1]-hept-5-en-3-one (1) was suspended in phosphate buffer (5 parts) and 50 mg of wet cell mass of culture (ATCC No.21285) was added and kept stirring 72 hr. The cell mass was removed by filtering through celite and the filtrate was extracted with dichloromethane (5 x 10 parts). Concentration of solvent gave optically active III in 31.8% chemical yield, and 58.2% ee.

### Example 3

General procedure for enantioselective hydrolysis of (±)-2-azabicyclo(2,2,1)hept-5-en-3-one, using cell mass from culture (ATCC No.21285). 0.1 g (0.00092 mole parts) of (±) was suspended in phosphate buffer (5 parts) and different amount of cell mass (as indicated in Table 1) was added and kept stirring 24 hr. The cell mass was removed by filtering through celite and the filtrate was extracted with dichloromethane (5x10 parts). Concentration of solvent gave optically active III. The results are summarized in Table 1.

**Table 1 .**

| **Sr.No.** | **Cells Wet/Wt.%** | **Chemical Yield** | **Ratio R:S** | **ee %** |
|---|---|---|---|---|
| 1. | 5 | 39.1 | 46.36:53.64 | 7.29 |
| 2. | 10 | 33.2 | 45.15:54.85 | 9.71 |
| 3. | 20 | 31.4 | 36.70:63.30 | 26.61 |
| 4. | 30 | 33.0 . | 27.43:72.57 | 45.14 |
| 5. | 40 | 34.2 | 25.71:74.29 | 48.58 |
| 6. | 50 | 30.5 | 21.23:78.77 | 57.55 |

### Example 4

General procedure for enantioselective hydrolysis of (±)2-azabicyclo(2,2,1)hept-5-en-3-one, using cell mass from culture (ATCC No.21285). 0.2 g (0.00184 mole parts) of(±) was suspended in phosphate buffer and organic solvent, (as indicated in Table 2) 10 parts, 0.1 gm of wet cell mass was added and kept stirring 24 hrs. The cell mass was removed by filtering through celite and the filtrate was extracted with dichloromethane (5 x 10 parts). Concentration of solvent gave optically active III. The results are summarized in Table 2.

**Table 2**

| **Sr.No.** | **Organic Solvent** | **Chemical Yield** | **Ratio R:S** | **ee %** |
|---|---|---|---|---|
| 1. | Ethyl acerate | 27.1 | 13,39 ; 86.61 | 73.22 |
| 2. | Methanol | 25.5 | 14.90 ; 85.10 | 70.20 |
| 3. | Ethanol | 33.1 | 9.27 ; 90.73 | 81.46 |
| 4. | Acetone | 44.2 | 9.69 ; 90.31 | 80.62 |
| 5. | Dimethyl sulfoxide | 42.1 | 36.10 : 63.90 | 27.80 |

The ratio of phosphate buffer (0.2M, pH 7.4) to organic solvent is (9:1).

### Example 5

General procedure for enantioselective hydrolysis of (±)2-azabicyclo(2,2,1)hept-5-en-3-one, using cell mass from culture (ATCC No.21285). 0.2 g (0.00184 mole parts) of(±) was suspended in phosphate buffer and acetone (as indicated in Table 2) 10 parts, 0.1 gm of wet cell mass was added and kept stirring 24 hrs. The cell mass was removed by filtering through celite and the filtrate was extracted with dichloromethane (5 x 10 parts). Concentration of solvent gave optically active III. The results of different proportions of acetone are summarized in Table 3.

**Table 3**

| **Sr.No.** | **Buffer : Acetone(v/v)** | **Chemical Yield** | **Ratio R:S** | **ee %** |
|---|---|---|---|---|
| 1. | 9.5 : 05 | 40.8 | 4.58 : 95.85 | 90.85 |
| 2. | 9.0 :1,0 | 41-2 | 9.69:90.31 | 80.62 |
| 3. | 8.0 : 2.0 | 41.3 | 12-34: 87.66 | 75.33 |
| 4. | 5.0 : 5.0 | Slow reaction | - | - |

### Example 6

General procedure for enantioselective hydrolysis of (±)2-azabicyclo(2,2,1)hept-5-en-3-one, using cell mass from culture (ATCC No.21285). 10.0 g (0.918 mole parts) of (±) was suspended in mixture of 475 parts of phosphate buffer and 25 parts of acetone in Lt. flask. Cell mass (wet. Weight - 5 parts) was added and the reaction mixture was stirred at room temperature (28±1). After completion of the reaction (monitored by chiral HPLC) the reaction mixture was centrifuged in order to remove cell mass and supernatent liquid was extracted using continuous extractor by dichloromethane. On evaporation of solvent under reduced pressure 3.93g of product was obtained. Crystallisation with dichloromethane : ether mixture gave a product of 98% optical purity.

## Claims

1. A process for the preparation of an optically active azabicylco heptanone derivative of the general Formula III, wherein R₁ = H, X = CH₂ Y-Z = -CH=CH- 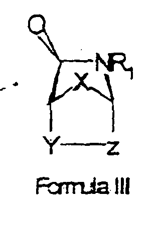 which comprises contacting (±)2-aza-bicyclo[2,2,1]hept-5-en-3-one of Formula I 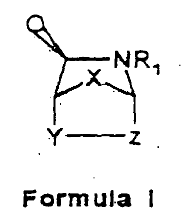 Wherein R₁=H, COR₂ (R₂=C₁₋₄ alkyl, C₁₋₄ alkoxy, aryl, aryloxy)
X=O, CHR₃ (R₃=F,OH,Br,H)
Y-Z=-CH=CH-,CH₂-CH₂, 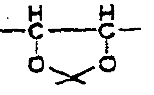 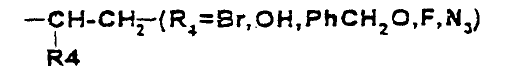 with whole cell *Kluyvera Citrophila* (ATCC No. 21285) or with a cell extract or lactamase enzyme extracted from *Kluyvera Citrophila* (ATCC No. 21285), in a buffer containing organic solvent, extracting the mixture with an organic solvent, separating the organic layer and removing the solvent to obtain the product.

2. A process as claimed in claim 1, wherein the buffer used is selected from the group consisting of phosphate buffer (0.05 M - 0.1 M, 6 - 8 pH), citrate buffer (0.05 M - 0.1 M, 6 - 7.5 pH) and Tris buffer (0.05 M - 0.2 M, 7 - 8 pH).

3. A process as claimed in claim 1, wherein the buffer used comprises phosphate buffer (0.2 M, 7.4 pH).

4. A process as claimed in any of claims 1 to 3, wherein the organic solvent used along with buffer is selected from the group consisting of alcohols, alkyl acetates, ketones and sulfoxides.

5. A process as claimed in claim 4, wherein the organic solvent is selected from the group consisting of methanol, ethanol, butanol, ethyl acetate, acetone, dimethyl sulfoxide and dimethyl formamide.

6. A process as claimed in claim 4, wherein the organic solvent comprises acetone.

7. A process as claimed in any preceding claim, wherein the percentage of organic solvent used for the reaction along with buffer is in the range 5% to 50% (v/v).

8. A process as claimed in claim 7, wherein the percentage of organic solvent used for the reaction along with buffer is 10% (v/v).

9. A process as claimed in any preceding claim, wherein the solvent used for extraction comprises a chlorinated solvent selected from the group consisting of chloroform, ethylene dichloride, methylene dichloride and an alkyl acetate.

10. A process as claimed in claim 9, wherein the alkyl acetate used for extraction comprises ethyl acetate.

11. A process as claimed in any of claims 1 to 8, wherein the solvent used for extraction comprises methylene chloride.

12. A process as claimed in any preceding claim, wherein the compound of Formula I is contacted with the enzyme or (whole) cell at a temperature in the range of 25-30°C.

13. A process as claimed in any preceding claim, wherein the compound of Formula I is contacted with the enzyme or (whole) cell for a period in the range 10 to 24 hours.

14. A process according to any preceding claim, wherein said enzyme is a lactamase enzyme or said cell is a cell expressing a lactamase enzyme.

## Patentansprüche

1. Verfahren zur Herstellung eines optisch aktiven Azabicycloheptanonderivats der allgemeinen Formel III, worin R₁ = H, X = CH₂, Y-Z = -CH=CH-, 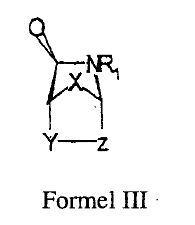 das die folgenden Verfahrensstufen umfaßt:
Umsetzen von (±)2-Azabicyclo[2,2,1]hept-5-en-3-on der Formel 1 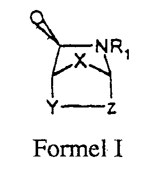 worin R₁=H, COR₂ (R₂=C₁₋₄ Alkyl, C₁₋₄ Alkoxy, Aryl, Aryloxy)
X=O, CHR₃ (R₃=F,OH,Br,H)
Y-Z=-CH=CH-,CH₂-CH₂, 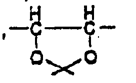 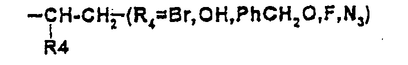
mit einer ganzen Zelle von *Kluyvera Citrophila* (ATCC Nr. 21285), oder einem Zellextrakt, oder mit einem aus *Kluyvera Citrophila* (ATCC Nr. 21285) extrahierten Lactamaseenzym in einem einen Puffer enthaltenden organischen Lösungsmittel;
Extrahieren des Gemisches mit einem organischen Lösungsmittel; Abtrennen der organischen Schicht; und
Entfernen des Lösungsmittels zwecks Erhalts des Produktes.

2. Verfahren nach Anspruch 1, wobei der verwendete Puffer ausgewählt wird aus der Gruppe bestehend aus einem Phosphatpuffer (0,05 M - 0,1 M, 6 - 8 pH), einem Citratpuffer (0,05 M-- 0,1 M, 6 - 7,5 pH) und einem Trispuffer (0,05 M - 0,2 M, 7-8 pH).

3. Verfahren nach Anspruch 1, wobei der verwendete Puffer ein Phosphatpuffer (0,2 M, 7,4 pH) ist.

4. Verfahren nach einem der Ansprüche 1 bis 3, wobei das zusammen mit dem Puffer verwendete organische Lösungsmittel ausgewählt wird aus der Gruppe bestehend aus Alkoholen, Alkylacetaten, Ketonen und Sulfoxiden.

5. Verfahren nach Anspruch 4, wobei das organische Lösungsmittel ausgewählt wird aus der Gruppe bestehend aus Methanol, Ethanol, Butanol, Ethylacetat, Aceton, Dimethylsulfoxid und Dimethylformamid.

6. Verfahren nach Anspruch 4, wobei das organische Lösungsmittel Aceton ist.

7. Verfahren nach einem der vorhergehenden Ansprüche, wobei der Prozentgehalt des für die Reaktion zusammen mit dem Puffer verwendeten organischen Lösungsmittels im Bereich zwischen 5% und 50% (V/V) liegt.

8. Verfahren nach Anspruch 7, wobei der Prozentgehalt des für die Reaktion zusammen mit dem Puffer verwendeten organischen Lösungsmittels 10% (V/V) beträgt.

9. Verfahren nach einem der vorhergehenden Ansprüche, wobei das für die Extraktion verwendete Lösungsmittel ein chloriertes Lösungsmittel, ausgewählt aus der Gruppe bestehend aus Chloroform, Ethylendichlorid, Methylendichlorid und einem Alkylacetat, ist.

10. Verfahren nach Anspruch 9, wobei das für die Extraktion verwendete Alkylacetat Ethylacetat ist.

11. Verfahren nach einem der Ansprüche 1 bis 8, wobei das für die Extraktion verwendete Lösungsmittel Methylendichlorid ist.

12. Verfahren nach einem der vorhergehenden Ansprüche, wobei die Umsetzung der Verbindung der Formel I mit dem Enzym oder der (ganzen) Zelle bei einer Temperatur im Bereich zwischen 25 und 30° C erfolgt.

13. Verfahren nach einem der vorhergehenden Ansprüche, wobei die Umsetzung der Verbindung der Formel I mit dem Enzym oder der (ganzen) Zelle während einer Zeitdauer im Bereich von 10 bis 24 Stunden erfolgt.

14. Verfahren nach einem der vorhergehenden Ansprüche, wobei besagtes Enzym ein Lactamaseenzym oder besagte Zelle eine ein Lactamaseenzym exprimierende Zelle ist.

## Revendications

1. Procédé de préparation d'un dérivé optiquement actif de l'azabicycloheptanone de formule générale III, dans laquelle R₁ représente H, X représente CH₂, Y-Z représente -CH=CH- 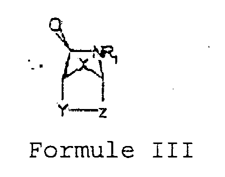 qui comprend la mise en contact de la (±)2-azabicyclo[2,2,1]hept-5-én-3-one de formule I 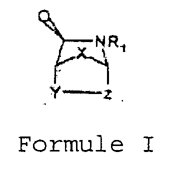 dans laquelle
R₁ représente H ou COR₂ (R₂ = alkyle en C₁-C₄, alkoxy en C₁-C₄, aryle, aryloxy)
X représente O, CHR₃ (R₃ = F, OH, Br, H)
Y-Z représente -CH=CH-, CH₂-CH₂, 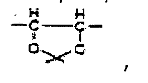 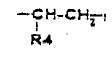 (R₄ = Br, OH, PhCH₂O, F, N₃)
avec une cellule entière de *Kluyvera Citrophila* (ATCC n°21285) ou avec un extrait cellulaire ou une enzyme lactamase extraite de *Kluyvera Citrophila* (ATCC n°21285), dans un tampon contenant un solvant organique, l'extraction du mélange avec un solvant organique, la séparation de la couche organique et l'élimination du solvant pour obtenir le produit.

2. Procédé tel que revendiqué dans la revendication 1, le tampon utilisé étant choisi dans le groupe comprenant le tampon phosphate (0,05 M-0, 1 M, pH 6-8), le tampon citrate (0,05 M-0,1 M, pH 6-7,5) et le tampon Tris (0,05 M-0,2 M, pH 7-8).

3. Procédé tel que revendiqué dans la revendication 1, le tampon utilisé comprenant le tampon phosphate (0,2 M, pH 7,4).

4. Procédé tel que revendiqué dans l'une quelconque des revendications 1 à 3, le solvant organique utilisé avec le tampon étant choisi dans le groupe comprenant les alcools, les acétates d'alkle, les cétones et les sulfoxydes.

5. Procédé tel que revendiqué dans la revendication 4, le solvant organique utilisé étant choisi dans le groupe comprenant le méthanol, l'éthanol, le butanol, l'acétate d'éthyle, l'acétone, le diméthylsulfoxyde et le diméthylformamide.

6. Procédé tel que revendiqué dans la revendication 4, le solvant organique utilisé comprenant l'acétone.

7. Procédé tel que revendiqué dans l'une quelconque des revendications précédentes, le pourcentage de solvant organique utilisé pour la réaction avec le tampon étant dans le domaine de 5% à 50% (v/v).

8. Procédé tel que revendiqué dans la revendication 7, le pourcentage de solvant organique utilisé pour la réaction avec le tampon étant de 10% (v/v).

9. Procédé tel que revendiqué dans l'une quelconque des revendications précédentes, le solvant utilisé pour l'extraction comprenant un solvant chloré choisi dans le groupe comprenant le chloroforme, le dichlorure d'éthylène, le dichlorure de méthylène et un acétate d'alkyle.

10. Procédé tel que revendiqué dans la revendication 9, l'acétate d'alkyle utilisé pour l'extraction comprenant l'acétate d'éthyle.

11. Procédé tel que revendiqué dans l'une quelconque des revendications 1 à 8, le solvant utilisé pour l'extraction comprenant le chlorure de méthylène.

12. Procédé tel que revendiqué dans l'une quelconque des revendications précédentes, le composé de formule I étant mis en contact avec l'enzyme ou la cellule (entière) à une température dans le domaine de 25 à 30°C.

13. Procédé tel que revendiqué dans l'une quelconque des revendications précédentes, le composé de formule I étant mis en contact avec l'enzyme ou la cellule (entière) pendant une durée dans le domaine de 10 à 24 heures.

14. Procédé tel que revendiqué dans l'une quelconque des revendications précédentes, ladite enzyme étant une enzyme lactamase ou ladite cellule étant une cellule exprimant une enzyme lactamase.
